# EUROPEAN PATENT APPLICATION

(11) **EP 4 613 316 A1**
(43) Date of publication of application: **10.09.2025**
(21) Application number: 24161973.3
(22) Date of filing: 07.03.2024
(51) Int. Cl.: A61M 25/00

(54) **A CATHETER FOR DELIVERING MEDICAL THERAPY TO A REMOTE SITE IN A BODY**

(71) Applicant: IMDS R&D BV, 9301 NZ Roden (NL)
(72) Inventor: SCHULTING, Edwin Alexander, Roden (NL)
(74) Representative: V.O.

(57) **Abstract**

A catheter comprises a tubular catheter wall, which comprises a skeleton wall section, which is at least extending between 30 mm and 4 mm proximal from the distal end of the catheter. The skeleton wall section comprises a skeleton (7), which has a distal skeleton end edge (6), which is extending in an endless manner all around a center line of the catheter wall. The distal skeleton end edge (6) is an undulated edge, which comprises N undulation tops (21, 22) and N undulation bottoms (31, 32). Axial distances (H) between each undulation top and each undulation bottom are at least 10% of an outer diameter (D) of the skeleton wall section. Said N undulation tops (21, 22), as well as said undulation bottoms (31, 32), are substantially equally angularly spaced relative to one another. The catheter exhibits improved navigational behaviour through bifurcations and/or tortuous pathways and/or stenoses.

## Description

The invention relates to a catheter for delivering medical therapy to a remote site in a body, wherein:
the catheter has a proximal end, a distal end and a longitudinal direction extending from the proximal end to the distal end;
the catheter comprises a tubular catheter wall, which is surrounding a lumen structure of the catheter;
the catheter wall comprises a skeleton wall section, which is extending in a skeleton range along the longitudinal direction; and
the skeleton wall section comprises:
   a skeleton, which is extending at least all along said skeleton range, and which provides reinforcement of the catheter wall in the longitudinal direction, as well in a circumferential direction around the longitudinal direction, wherein the skeleton is an interrupted tubular reinforcement wall obtained by microfabricating a cut pattern in a fully uninterrupted tubular reinforcement wall made of a reinforcement material,
   an inner liner, which is co-axially surrounded by said skeleton relative to a center line of the catheter wall, and
   an outer laminate, which is co-axially surrounding said skeleton relative to the center line of the catheter wall.

US 2019/0255290 A1 discloses an example of a catheter of the type as initially identified above. US 2019/0255290 A1 discloses in Fig. 3 an example of an interventional cardiovascular catheter specifically designed for being positioned near the aortic root. Figs. 4A-4D and Fig. 5 of US 2019/0255290 A1 show some examples of skeletons that can be applied in the various longitudinal sections of the catheter of Fig. 3 of US 2019/0255290 A1, wherein such a skeleton is an interrupted tubular reinforcement wall obtained by microfabricating a cut pattern in a fully uninterrupted tubular reinforcement wall made of a reinforcement material. The catheter of Fig. 3 of US 2019/0255290 A1 has been particularly designed for providing a relatively sharp bend 54 (see Fig. 2 of US 2019/0255290 A1) to extend across the aortic root 16 and reach into the artery 24 (see Fig. 2 of US 2019/0255290 A1). Especially the proximal-intermediate section 106 of Fig. 3 of US 2019/0255290 A1), which includes the one-beam configuration of the skeleton of Fig. 4D of US 2019/0255290 A1) has been designed for providing said relatively sharp bend 54. US 2019/0255290 A1 further dicloses that the catheter of Fig. 3 of US 2019/0255290 A1 may have an inner liner and/or an outer laminate which are extending distally beyond the skeleton to form an atraumatic distal tip 116 of the catheter.

As compared to the above-mentioned very specific design object of US 2019/0255290 A1 to improve positioning of a catheter near the aortic root, the present invention has a different object.

It is an object of the present invention to provide a solution for improving the navigational behaviour of catheters in a widely applicable manner through bifurcations and/or tortuous pathways and/or stenoses.

For that purpose the invention provides a catheter according to the appended independent claim 1. Preferable embodiments of the invention are provided by the appended dependent claims 2-10.

Hence, the invention provides a catheter for delivering medical therapy to a remote site in a body, wherein:
the catheter has a proximal end, a distal end and a longitudinal direction extending from the proximal end to the distal end;
the catheter comprises a tubular catheter wall, which is surrounding a lumen structure of the catheter, and which is extending from the distal end, proximally along the longitudinal direction, to at least 100 mm proximal from the distal end;
the catheter wall comprises a skeleton wall section, which is extending in a skeleton range along the longitudinal direction, wherein said skeleton range is at least extending between 30 mm proximal from the distal end and 4 mm proximal from the distal end;
the skeleton wall section comprises:
   a skeleton, which is extending at least all along said skeleton range, and which provides reinforcement of the catheter wall in the longitudinal direction, as well in a circumferential direction around the longitudinal direction, wherein the skeleton is an interrupted tubular reinforcement wall obtained by microfabricating a cut pattern in a fully uninterrupted tubular reinforcement wall made of a reinforcement material,
   an inner liner, which is co-axially surrounded by said skeleton relative to a center line of the catheter wall, and
   an outer laminate, which is co-axially surrounding said skeleton relative to the center line of the catheter wall;
   the skeleton has a distal skeleton end edge, which is extending in an endless manner all around the center line, said distal skeleton end edge being an undulated edge in that it comprises a number N of undulation tops and a number N of undulation bottoms, which are alternating with one another, wherein N ≥ 2, and wherein the distal skeleton end edge is facing with said N undulation tops distally along the longitudinal direction, and wherein the distal skeleton end edge is facing with said N undulation bottoms proximally along the longitudinal direction, and wherein each of said N undulation tops of the distal skeleton end edge is a round, flat or pointed top and each of said N undulation bottoms of the distal skeleton end edge is a round, flat or pointed bottom;
   for each concerned undulation top of said N undulation tops, all N axial distances measured along the longitudinal direction between the undulation top concerned and each of said N undulation bottoms, respectively, are at least 10% of an outer diameter of the skeleton wall section, said outer diameter being measured at 7 mm proximal from the distal end; and
   said undulation tops are angularly spaced relative to one another around the center line by an angular spacing of 360 degrees divided by N, with allowable deviations of said angular spacing in a deviation range in-between plus or minus 15% of 360 degrees divided by N, and wherein also said undulation bottoms are angularly spaced relative to one another around the center line by said angular spacing with said allowable deviations.

In the above-recited features of the present invention, the key features of the invention are:
i. that the skeleton is extending at least all along the skeleton range, which is extending at least between 30 mm proximal from the distal end and 4 mm proximal from the distal end;
ii. that the skeleton has the distal skeleton end edge, which is extending in an endless manner all around the center line, and which is an undulated edge having N undulation tops and N undulation bottoms, wherein N ≥ 2;
iii. that the recited N axial distances between undulation tops and undulation bottoms are at least 10% of the recited outer diameter; and
iv. the specific recited angular spacings of the N undulation tops and N undulation bottoms.

These combined key features (i), (ii), (iii) and (iv) of the skeleton provide the skeleton wall section with a specific gradual transition of the bending stiffness, from "relatively flexible" at the N undulation tops to "more stiff' at the N undulation bottoms. In use, the N undulation tops may flex radially inward as compared to the N undulation bottoms, which during navigation appears to provide the skeleton wall in the distal tip section of the catheter with a subtle dynamic tapering effect. This appears to provide surprising and remarkable advantages when navigating a catheter according to the invention through bifurcations and/or tortuous pathways and/or stenoses. It is noted that the flexibility of, especially, the distal tip section of a catheter is a determinative factor to successively navigate a catheter through bifurcations and/or tortuous pathways and/or stenoses. After all, once the distal tip has passed a challenging bifurcation, curve or stenosis, the remainder of the catheter generally follows.

Hence, in view of the above it will be appreciated that the present invention improves the navigational behaviour of catheters through bifurcations and/or tortuous pathways and/or stenoses.

In a preferable embodiment of a catheter according to the invention, for each concerned undulation top of said N undulation tops, all N axial distances measured along the longitudinal direction between the undulation top concerned and each of said N undulation bottoms, respectively, are at least 20% of said outer diameter, more preferably at least 30% of said outer diameter, and yet more preferably at least 35% of said outer diameter.

In another preferable embodiment of a catheter according to the invention, said deviation range more specifically is in-between plus or minus 10% of said angular spacing, and preferably in-between plus or minus 5% of said angular spacing, and more preferably in-between plus or minus 1% of said angular spacing. Ideally, there would be no such deviation range, so that said undulation tops, as well as said undulation bottoms are equally angularly spaced relative to one another around the center line by an angular spacing of 360 degrees divided by N. Such an equal angular spacing gives omni-directionally well-distributed favourable bending stiffness behaviour for navigating catheters through bifurcations and/or tortuous pathways and/or stenoses.

In another preferable embodiment of a catheter according to the invention, said skeleton range is at least extending between 30 mm proximal from the distal end and 3 mm proximal from the distal end, more preferably between 30 mm proximal from the distal end and 2 mm proximal from the distal end.

In another preferable embodiment of a catheter according to the invention an overall skeleton cut-away ratio of a longitudinal section of the catheter wall in a longitudinal range along the longitudinal direction is defined as the percentage of:
- the volume of all parts, within said longitudinal range, of said reinforcement material being cut-away from said fully uninterrupted tubular reinforcement wall according to said microfabricated cut pattern,
   relative to
- the volume of all parts, within said longitudinal range, of said reinforcement material of said fully uninterrupted tubular reinforcement wall; and
the overall skeleton cut-away ratio of the skeleton wall section in said skeleton range is between 40% and 90%, more preferably between 60% and 90%.

Said high overall skeleton cut-away ratio of at least 40% (more preferably at least 60%) of the skeleton wall section further contributes to obtaining a favourable bending stiffness of the skeleton wall section for navigating a catheter according to the invention through bifurcations and/or tortuous pathways and/or stenoses.

It is noted that manufacturing said high overall skeleton cut-away ratio of at least 40% (more preferably at least 60%) of the skeleton wall section according to the invention requires inventive measures to solve several problems. The reason is that the inner liner of a catheter having a skeleton for many applications typically is a tube made of, for example, PTFE (polytetrafluoroethylene, e.g. Teflon^{®}), PVDF (polyvinylidene fluoride, e.g. Kynar^{®}) or HDPE (high-density polyethylene), which during manufacturing of the catheter is axially inserted into the skeleton, and which is then expanded against the skeleton by inflation and heat. For said high cut-away ratios the problems arise that the tube made of, for example, PTFE, PVDF or HDPE being inflated against the skeleton will bulge out of the large cut-outs in the skeleton material and/or will break under the inflation pressure. Another problem, especially for skeletons made of less strong material, is that the skeleton may severely deform and/or crack and/or break under the inflation pressure due to the fact that the skeleton is severely weakened by the large cut-outs in the skeleton material.

In connection therewith, the present disclosure inter alia is based on the new insights, as disclosed herein, that said problems can be solved by introducing, temporarily during the manufacturing of a catheter according to the invention, a heat-shrinkable outer tube over the outer side of the skeleton, so that the heat-shrinkable outer tube temporarily closes off the cut-outs in the skeleton material during said expansion of the inner liner by inflation and heat, while at the same time the heat-shrinkable outer tube temporarily reinforces the skeleton during said expansion of the inner liner by inflation and heat. After the inner liner has been successfully expanded against the skeleton and after the catheter under construction has been cooled, the temporary heat-shrinkable outer tube can be removed from the outer side of the skeleton, and be replaced by the final outer laminate over the outer side of the skeleton.

Alternatively, another new insight, as disclosed herein, is that said problems can be solved by performing the following manufacturing steps for assembling the inner liner and the outer laminate to the skeleton in case of said high overall skeleton cut-away ratio (at least 40%, more preferably at least 60%) of the skeleton wall section of the catheter wall of a catheter according to the invention. Firstly, a tubular base layer of fluorinated ethylene propylene (FEP) is extruded and the inner liner is extruded over the base layer. Next, the FEP base layer together with the inner liner is axially inserted into the skeleton. Next, the outer laminate is slided over the outer side of the skeleton. Thereafter, a temporary heat-shrinkable outer FEP tube is slided over the outer laminate, and the total assembly is heated. During heating the temporary heat-shrinkable outer FEP tube provides high compression onto the assembly of the inner liner, the skeleton and the outer laminate. After the catheter under construction has been cooled, the temporary heat-shrinkable outer tube can be removed from the outer side of the skeleton, and the FEP base layer can be axially pulled out of the assembly, making use of the favourable properties of FEP with respect to being pulled. Thus, a very close fit of the inner liner against the inner side of the skeleton is obtainable.

It is noted that the last-mentioned use of such an extruded FEP base layer is a more effective and much less expensive alternative as compared to the traditional use of a temporary silver plated copper wire, which at the end of a manufacturing sub-process is axially pulled out of an inner liner of a catheter under construction.

In another preferable embodiment of a catheter according to the invention said distal skeleton end edge is an edge of an elongated undulated strut structure, which is a part of the skeleton, and which is extending in an endless manner all around the center line. Such an elongated undulated strut structure, which is a part of the skeleton, and which is extending in an endless manner all around the center line, further improves the above-mentioned specific gradual transition of the bending stiffness of the skeleton wall section and the above-mentioned subtle dynamic tapering effect when navigating a catheter according to the invention through bifurcations and/or tortuous pathways and/or stenoses.

In another preferable embodiment of a catheter according to the invention, a radiopaque element is embedded in the catheter wall in that the radiopaque element is located in a space of the cut pattern, said space being bounded by the skeleton, the inner layer and the outer laminate. This is an efficient way of embedding a radiopaque element (e.g. a radiopaque marker) in the catheter wall, since the radiopaque element, can be perfectly located in such a space of the cut pattern.

The catheter according to the invention may be embodied as a rapid exchange catheter having the tubular catheter wall as a distal rapid exchange tubular segment which is spaced from the proximal end of the rapid exchange catheter. Such a rapid exchange catheter has a proximal catheter segment, which is lying proximally from the distal rapid exchange tubular segment, and which may, for example, be a push rod which is connected to the distal rapid exchange tubular segment. Such a push rod may advantageously have a round design with a diameter ≤ 0.40 mm to support 1:1 torquability from the proximal end to the distal end. Such a 1:1 torquability is highly desirable for this kind of device as the physician needs to be able to precisely steer the catheter towards a target site, such as a bend or a side branch of a vasculature passageway.

Alternatively, the catheter according to the invention may be embodied as an over-the-wire (OTW) catheter, wherein the tubular catheter wall and the lumen structure of the catheter are extending all along the longitudinal direction from the proximal end to the distal end. Such an over-the-wire catheter may advantageously have an over-the-wire catheter skeleton, which is extending all along the longitudinal direction from the proximal end to the distal end, and which provides reinforcement of the catheter wall in the longitudinal direction, as well in the circumferential direction around the longitudinal direction, wherein the over-the-wire catheter skeleton is an interrupted tubular reinforcement wall obtained by microfabricating a cut pattern in a fully uninterrupted tubular reinforcement wall made of a reinforcement material. Hence, in this case, the skeleton of the skeleton wall section is a section of the over-the-wire catheter skeleton. Preferably the over-the-wire catheter skeleton is designed to support 1:1 torquability from the proximal end to the distal end of the over-the-wire catheter. Alternatively to said over-the wire skeleton, the over-the-wire catheter may, in longitudinal sections outside the skeleton range of the skeleton wall section, also be designed with, for example, braids or wire coil structures to support the 1:1 torquability.

In the following, the invention is further elucidated with reference to nonlimiting embodiments and with reference to the schematic figures in the appended drawing, in which the following is shown.
Fig. 1 shows, in a longitudinal side view, an example of an embodiment of a catheter according to the invention, wherein in Fig. 1 the skeleton range of the skeleton wall section of the catheter wall has been indicated by a brace mark, and wherein in the embodiment of the catheter of Fig. 1 the above-mentioned number N is equal to two (N = 2).
Fig. 2 shows, in a perspective view, a longitudinal section of the skeleton of the skeleton wall section of the catheter of Fig. 1.
Fig. 3A shows a short longitudinal section of the skeleton wall section of the catheter of Fig. 1, wherein the shown longitudinal section comprises a part of the skeleton, and wherein the shown view is a longitudinal sectional view, which contains the center line of the catheter wall.
Fig. 3B shows a cross-section through the longitudinal section of the skeleton wall section of Fig. 3A, wherein the shown cross-section is perpendicular to the center line of the catheter wall.
Fig. 4 shows a short longitudinal section of the skeleton of the skeleton wall section of the catheter of Fig. 1, wherein the shown view is a top view on the longitudinal section in an imaginary "unrolled tube" state of the longitudinal section.
Fig. 5 shows, in a view similar to the view of Fig. 4, a short longitudinal section of an example of an alternative embodiment of a skeleton of a catheter according to the invention, wherein in the alternative embodiment of the skeleton of Fig. 5 the above-mentioned number N is equal to three (N = 3).

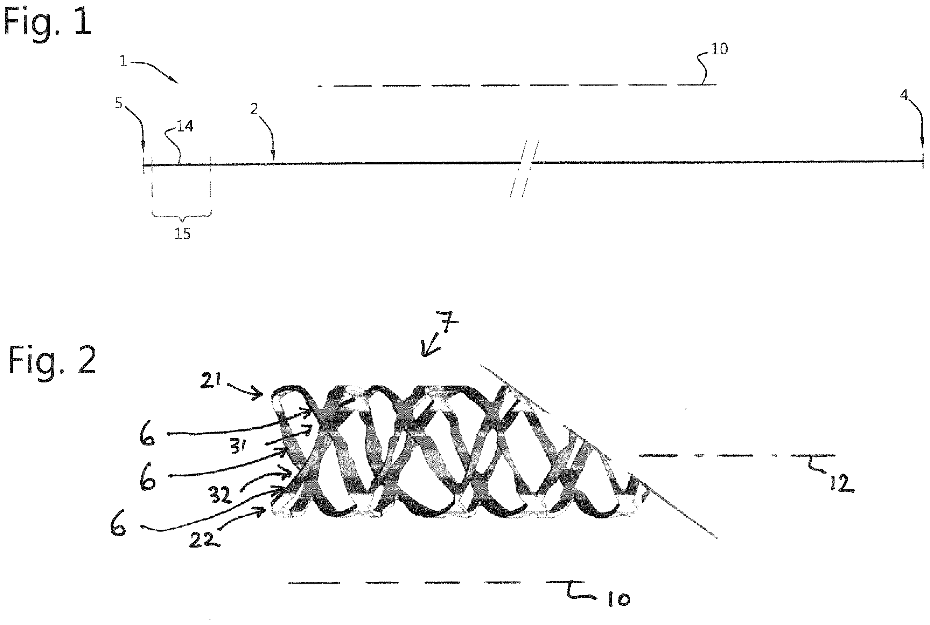

The reference signs used in the embodiment of Figs. 1-4 are referring to the above-mentioned parts and aspects of the invention, as well as to related parts and aspects, in the following manner.
- 1: catheter
- 2: tubular catheter wall
- 3: lumen structure
- 4: proximal end
- 5: distal end
- 6: distal skeleton end edge
- 7: skeleton
- 7 A: cut pattern
- 8: inner liner
- 9: outer laminate
- 10: longitudinal direction
- 11: circumferential direction
- 12: center line
- 14: skeleton wall section
- 15: skeleton range
- 21, 22: two undulation tops
- 31, 32: two undulation bottoms
- 91-95: five connection nodes of the skeleton 7
- D: outer diameter
- H: axial distance

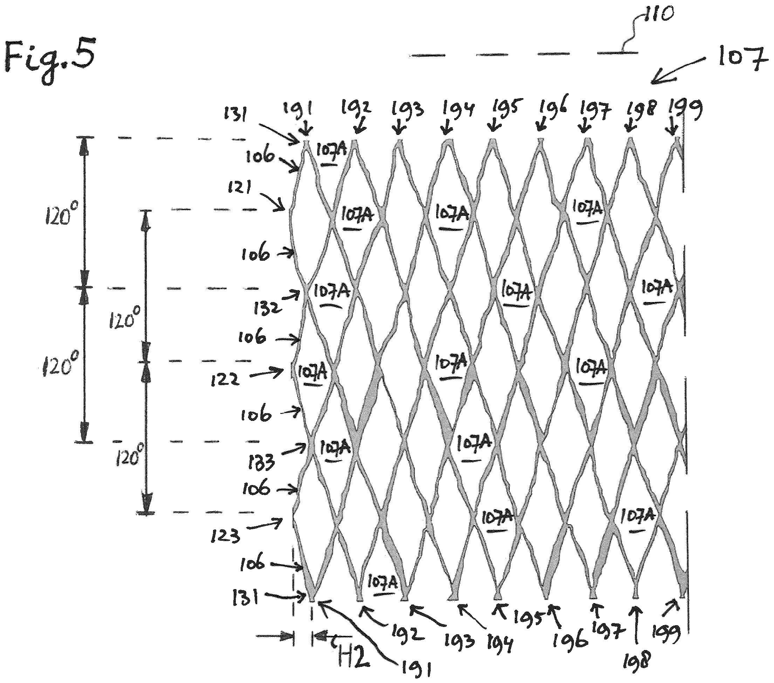

The reference signs used in the alternative embodiment of Fig. 5 are referring to the above-mentioned parts and aspects of the invention, as well as to related parts and aspects, in the following manner.
- 106: distal skeleton end edge
- 107: skeleton
- 107A: cut pattern
- 110: longitudinal direction
- 121, 122, 123: three undulation tops
- 131, 132, 133: three undulation bottoms
- 191-199: nine connection nodes of the skeleton 107
- H2: axial distance

Based on the above introductory description, including the brief description of the drawing figures, and based on the above-listed reference signs used in Figs. 1-5, the embodiment of Figs. 1-4 and the alternative embodiment of Fig. 5 are for the greatest part readily self-explanatory. The following extra explanations are given.

Fig. 1 shows the catheter 1 having the longitudinal direction 10, the proximal end 4 and the distal end 5. Fig. 1 further shows the skeleton range 15 of the skeleton wall section 14 of the catheter wall 2.

Fig. 2 shows a longitudinal section of the skeleton 7 of the skeleton wall section 14 of the catheter 1 of Fig. 1. In the shown example, the skeleton 7 is formed by a tubular lattice of elongated skeleton struts, which are interconnected with one another at a multiplicity of connections nodes of the skeleton 7.

The distal skeleton end edge 6 of the skeleton 7 is shown on the left-hand side of Fig. 2. In the shown example, the distal skeleton end edge 6 is an edge of an elongated undulated strut structure, which is a part of the skeleton 7, and which is extending in an endless manner all around the center line 12 of the catheter wall 2.

As mentioned, the embodiment of Figs. 1-4 is an embodiment in which N = 2. In line therewith, Fig. 2 shows that the distal skeleton end edge 6 of the skeleton 7 of the skeleton wall section 14 of the catheter 1 of Fig. 1 has the two undulation tops 21 and 22, as well as the two undulation bottoms 31 and 32.

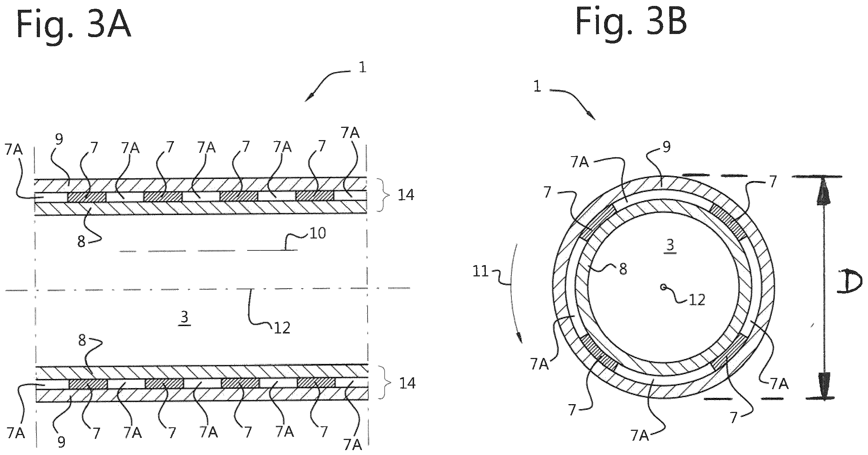

Figs. 3A-3B show that the skeleton wall section 14 not only has the skeleton 7, which comprises the cut pattern 7A, but also has the inner liner 8 and the outer laminate 9. In the example of Figs. 3A-3B, the lumen structure 3 of the catheter 1 is a single lumen structure. Alternatively, a catheter according to the invention may have a multiple lumen structure.

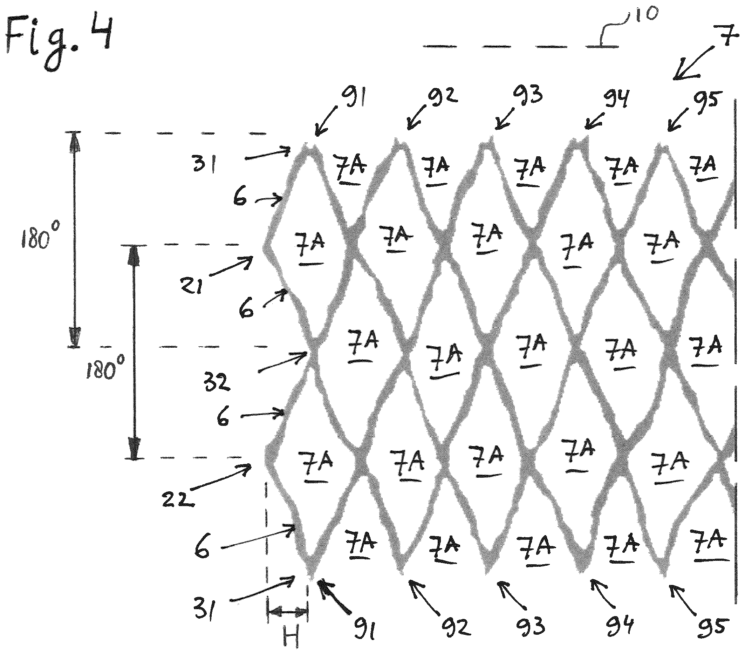

Fig. 4 shows a short longitudinal section of the skeleton 7 of the skeleton wall section 14 of the catheter 1. In Fig. 4, the shown view is a top view on the longitudinal section in an imaginary "unrolled tube" state of the longitudinal section. To explain the term "unrolled tube" it is noted that the skeleton 7 is a tubular wall (more particularly an interrupted tubular reinforcement wall), and that such a tubular wall imaginarily can be unrolled into a completely straight state by imaginarily unrolling it onto a fully straight plane after an imaginary cut, substantially parallel to the center line 12 of the catheter 1, has been made over the full length of the tubular wall. In Fig. 4 said imaginary straight cut is an imaginary line that passes through the connection nodes 91, 92, 93, 94 and 95 of the skeleton 7. In Fig. 4 said reference numerals 91-95 are shown both in the lower part of Fig. 4 and in the upper part of Fig. 4, since the locations to which these reference numerals 91-95 are pointing will join when the completely straight state of Fig. 4 would be "rolled up" again to form the tubular state of the skeleton 7 as shown in the perspective view of Fig. 2.

Fig. 4 also shows the two undulation tops 21 and 22, as well as the two undulation bottoms 31 and 32 of Fig. 2. It is noted that in Fig. 4 the reference numeral of the undulation bottom 31 is shown both in the lower part of Fig. 4 and in the upper part of Fig. 4. This is done for the reason that the two locations to which the reference numeral 31 is pointing will join when the completely straight state of Fig. 4 would be "rolled up" again to form the tubular state of the skeleton 7 as shown in the perspective view of Fig. 2.

In Fig. 4 the reference sign H indicates the axial distance between the undulation top 22 and the undulation bottom 31. In the shown example, this axial distance H is slightly higher than 30% of the outer diameter D (indicated in Fig. 3B) of the skeleton wall section, wherein said outer diameter D is measured at 7 mm proximal from the distal end 5. Furthermore, in Fig. 4 also the axial distance between the undulation top 22 and the other undulation bottom 32 is slightly higher than 30% of the outer diameter D, while also the axial distance between the other undulation top 21 and the undulation bottom 31, as well as the axial distance between the undulation top 21 and the other undulation bottom 32 are each slightly higher than 30% of the outer diameter D. Accordingly, the embodiment of Figs. 1-4 is an example of the above-mentioned preferable embodiment in which "for each concerned undulation top of said N undulation tops, all N axial distances measured along the longitudinal direction between the undulation top concerned and each of said N undulation bottoms, respectively, are at least 20% of said outer diameter, more preferably at least 30% of said outer diameter".

Furthermore, from the 180 degrees' indications in Fig. 4 it follows that the undulation tops 21 and 22 are equally angularly spaced relative to one another around the center line 12 by an angular spacing of 180 degrees, which is 360 degrees divided by N, wherein N = 2. Similarly it follows from Fig. 4 that the undulation bottoms 31 and 32 are equally angularly spaced relative to one another around the center line 12 by an angular spacing of 180 degrees.

Reference is now made to Fig. 5, which shows a short longitudinal section of the skeleton 107 as an alternative embodiment to the skeleton 7 of Figs. 2-4. The view of Fig. 5 is similar to that of Fig. 4. Hence, it is a top view on the longitudinal section in the above-explained imaginary "unrolled tube" state. In the alternative skeleton 107 of Fig. 5 the above-mentioned number N is equal to three (N = 3). In line therewith, Fig. 5 shows the three undulation tops 121, 122 and 123, as well as the three undulation bottoms 131, 132 and 133.

In Fig. 5 the reference sign H2 indicates the axial distance between the undulation top 123 and the undulation bottom 131. In the shown example, this axial distance H2 is slightly higher than 10% of the outer diameter of the skeleton wall section, said outer diameter being measured at 7 mm proximal from the distal end 5. More particularly, in Fig. 5 for each concerned undulation top of the three undulation tops 121, 122 and 123, all three axial distances measured along the longitudinal direction between the undulation top concerned and each of the three undulation bottoms 131, 132 and 133, respectively, are at least 10% of the outer diameter of the skeleton wall section, said outer diameter being measured at 7 mm proximal from the distal end 5.

Furthermore, from the 120 degrees' indications in Fig. 5 it follows that the undulation tops 121, 122 and 123 are equally angularly spaced relative to one another around the center line of the catheter wall by an angular spacing of 120 degrees, which is 360 degrees divided by N, wherein N = 3. Similarly it follows from Fig. 5 that the undulation bottoms 131, 132 and 133 are equally angularly spaced relative to one another around the center line of the catheter wall by an angular spacing of 120 degrees.

By using the examples of each of the skeletons 7 and 107 shown in Figs. 4 and 5, respectively, catheters can be obtained according to the above-mentioned preferable embodiments of the invention in which the overall skeleton cut-away ratio of the skeleton wall section in the skeleton range is between 40% and 90%, and more specifically between 60% and 90%.

A tubular catheter wall, which comprises the skeleton 7 with its distal skeleton end edge 6, or which comprises the skeleton 107 with its distal skeleton end edge 106, may for example be part of a rapid exchange catheter having the tubular catheter wall as a distal rapid exchange tubular segment which is spaced from the proximal end of the rapid exchange catheter. Alternatively, such a tubular catheter wall may for example be part of an over-the-wire (OTW) catheter, wherein the tubular catheter wall is extending all along the longitudinal direction from the proximal end to the distal end.

From the above-explained example embodiments of a catheter according to the invention for N = 2 and N = 3, respectively, it will be sufficiently clear to the reader of the present disclosure how examples of embodiments of a catheter according to the invention for N ≥ 4 can be designed analogously.

It is remarked that in the shown examples of the two skeletons 7 and 107, respectively, each of these two skeletons is a "randomized skeleton" in the sense that it comprises a plurality of interconnected struts, wherein, as seen in radial view on the randomized skeleton, said radial view being radially in a cylindrical coordinate system relative to the center line:
- each of said struts has a corresponding pair of two mutually opposing longitudinal strut edges alongside one another, which are determining a corresponding distribution of tangential thickness of a strut concerned longitudinally along the strut concerned, said tangential thickness being tangentially in a cylindrical coordinate system relative to the center line,
- said longitudinal strut edges are bordering the cut pattern (7A or 107A in Figs. 4 and 5, respectively), and
- each of said longitudinal strut edges has random bends and/or random cusps.

Such "randomly shaped" skeleton strut edges not only provide improved mutual bondings between the skeleton, inner liner and outer laminate, but also provide improved torquability of the catheter, including a preventive effect on the occurrence of the so-called "whip" phenomenon during torquing.

Such "randomized skeletons" having such "randomly shaped" skeleton strut edges are described in European patent application no. 23168923.3, which was filed on 20 April 2023, and which has the same inventor as the inventor of the present invention of the present disclosure. The present invention, however, is not limited to "randomized skeletons" having such "randomly shaped" skeleton strut edges.

In fact, the present invention can be practiced with many various structures, many various materials, many various shapes and many various dimensions of the skeleton, inner liner and outer laminate of the catheter, and with many various additional features and many various accessories of the catheter, such as the many various structures, the many various materials, the many various shapes and the many various dimensions of skeletons, inner liners and outer laminates, and the many various additional features and the many various accessories that are used in known catheters for delivering medical therapy to a remote site in a body, as long as the catheter is within the scope of the appended claims.

Regarding the overall skeleton cut-away ratio the following general remark is made. If, everywhere in a longitudinally continuous longitudinal section of a catheter wall, the catheter wall has a totally uninterrupted tubular metal reinforcement wall, for example in the form of a totally uninterrupted metal hypotube, then said longitudinal section has an overall skeleton cut-away ratio of 0%.

It is further noted that according to the invention typical dimensions of some parts and aspects of the invention, and applicable practical ranges of such dimensions could be as follows in case the catheter is a microcatheter for interventional cardiovascular or neurovascular applications. Effective length of the catheter (as measured from the distal end of a hub of the catheter) can be in the range between 130 cm and 160 cm, and can typically be 135 cm. Maximum outer diameter of the skeleton wall section of the tubular catheter wall of the catheter can be in the range between 0.60 mm and 1.20 mm, and can typically be 0.75 mm, while noting that the word "maximum" in the term "maximum outer diameter" is referring to the maximum value as considered over the full length of the skeleton wall section. Minimum inner diameter of the skeleton wall section of the tubular catheter wall of the catheter can be in the range between 0.36 mm and 0.55 mm, and can typically be 0.45 mm, while noting that the word "minimum" in the term "minimum inner diameter" is referring to the minimum value as considered over the full length of the skeleton wall section. For example, the following typical combinations of maximum outer diameter and minimum inner diameter of the skeleton wall section are possible: maximum outer diameter 0.75 mm in combination with minimum inner diameter 0.45 mm, maximum outer diameter 1.20 mm in combination with minimum inner diameter 0.55 mm, or maximum outer diameter 0.60 mm in combination with minimum inner diameter 0.36 mm.

However, the present invention can be embodied in various other catheters for delivering medical therapy to a remote site in a body, which can be non-vascular and/or non-micro catheters, such as for example catheters for delivering medical therapy to renal vessels, fallopian tubes, and other such vessels and sites. In such cases typical dimensions of some parts and aspects of the invention, and applicable practical ranges of such dimensions may be different from those mentioned above for microcatheters for interventional cardiovascular or neurovascular applications.

## Claims

1. A catheter (1) for delivering medical therapy to a remote site in a body, wherein:
the catheter has a proximal end (4), a distal end (5) and a longitudinal direction (10) extending from the proximal end to the distal end;
the catheter comprises a tubular catheter wall (2), which is surrounding a lumen structure (3) of the catheter, and which is extending from the distal end (5), proximally along the longitudinal direction, to at least 100 mm proximal from the distal end;
the catheter wall (2) comprises a skeleton wall section (14), which is extending in a skeleton range (15) along the longitudinal direction, wherein said skeleton range is at least extending between 30 mm proximal from the distal end (5) and 4 mm proximal from the distal end (5);
the skeleton wall section (14) comprises:
- a skeleton (7), which is extending at least all along said skeleton range, and which provides reinforcement of the catheter wall (2) in the longitudinal direction (10), as well in a circumferential direction (11) around the longitudinal direction (10), wherein the skeleton (7) is an interrupted tubular reinforcement wall obtained by microfabricating a cut pattern (7A) in a fully uninterrupted tubular reinforcement wall made of a reinforcement material,
- an inner liner (8), which is co-axially surrounded by said skeleton (7) relative to a center line (12) of the catheter wall (2), and
- an outer laminate (9), which is co-axially surrounding said skeleton (7) relative to the center line (12) of the catheter wall;
the skeleton (7) has a distal skeleton end edge (6), which is extending in an endless manner all around the center line (12), said distal skeleton end edge being an undulated edge in that it comprises a number N of undulation tops (21, 22) and a number N of undulation bottoms (31, 32), which are alternating with one another, wherein N ≥ 2, and wherein the distal skeleton end edge (6) is facing with said N undulation tops (21, 22) distally along the longitudinal direction (10), and wherein the distal skeleton end edge (6) is facing with said N undulation bottoms (31, 32) proximally along the longitudinal direction (10), and wherein each of said N undulation tops (21, 22) of the distal skeleton end edge (6) is a round, flat or pointed top and each of said N undulation bottoms (31, 32) of the distal skeleton end edge is a round, flat or pointed bottom;
for each concerned undulation top of said N undulation tops (21, 22), all N axial distances (H) measured along the longitudinal direction (10) between the undulation top concerned and each of said N undulation bottoms (31, 32), respectively, are at least 10% of an outer diameter (D) of the skeleton wall section (14), said outer diameter (D) being measured at 7 mm proximal from the distal end (5); and
said undulation tops (21, 22) are angularly spaced relative to one another around the center line (12) by an angular spacing of 360 degrees divided by N, with allowable deviations of said angular spacing in a deviation range in-between plus or minus 15% of 360 degrees divided by N, and wherein also said undulation bottoms (31, 32) are angularly spaced relative to one another around the center line (12) by said angular spacing with said allowable deviations.

2. The catheter (1) according to claim 1, wherein for each concerned undulation top of said N undulation tops (21, 22), all N axial distances (H) measured along the longitudinal direction (10) between the undulation top concerned and each of said N undulation bottoms (31, 32), respectively, are at least 20% of said outer diameter (D), and preferably at least 30% of said outer diameter (D), and more preferably at least 35% of said outer diameter (D).

3. The catheter (1) according to claim 1 or 2, wherein said deviation range more specifically is in-between plus or minus 10% of said angular spacing, and preferably in-between plus or minus 5% of said angular spacing, and more preferably in-between plus or minus 1% of said angular spacing.

4. The catheter (1) according to any one of the preceding claims, wherein said skeleton range (15) is at least extending between 30 mm proximal from the distal end (5) and 3 mm proximal from the distal end (5), and preferably between 30 mm proximal from the distal end (5) and 2 mm proximal from the distal end (5).

5. The catheter (1) according to any one of the preceding claims, wherein an overall skeleton cut-away ratio of a longitudinal section of the catheter wall (2) in a longitudinal range along the longitudinal direction (10) is defined as the percentage of:
- the volume of all parts, within said longitudinal range, of said reinforcement material being cut-away from said fully uninterrupted tubular reinforcement wall according to said microfabricated cut pattern (7A), relative to
- the volume of all parts, within said longitudinal range, of said reinforcement material of said fully uninterrupted tubular reinforcement wall; and
the overall skeleton cut-away ratio of the skeleton wall section (14) in said skeleton range (15) is between 40% and 90%.

6. The catheter (1) according to claim 5, wherein the overall skeleton cut-away ratio of the skeleton wall section (14) in said skeleton range (15) is between 60% and 90%.

7. The catheter (1) according to any one of the preceding claims, wherein said distal skeleton end edge (6) is an edge of an elongated undulated strut structure, which is a part of the skeleton (7), and which is extending in an endless manner all around the center line (12).

8. The catheter (1) according to any one of the preceding claims, wherein a radiopaque element is embedded in the catheter wall (2) in that the radiopaque element is located in a space of the cut pattern (7A), said space being bounded by the skeleton (7), the inner layer (8) and the outer laminate (9).

9. The catheter (1) according to any one of the preceding claims, wherein the catheter is a rapid exchange catheter having the tubular catheter wall (2) as a distal rapid exchange tubular segment which is spaced from the proximal end (4) of the rapid exchange catheter.

10. The catheter (1) according to any one of claims 1-8, wherein the catheter is an over-the-wire (OTW) catheter, wherein the tubular catheter wall (2) and the lumen structure (3) of the catheter are extending all along the longitudinal direction (10) from the proximal end (4) to the distal end (5).
